# EUROPEAN PATENT SPECIFICATION

(11) **EP 3 607 943 B1**
(45) Date of publication and mention of the grant of the patent: **11.09.2024**
(21) Application number: 19194585.6
(22) Date of filing: 03.04.2012
(51) Int. Cl.: A61K 31/137, A61K 9/00, A61K 9/20, A61P 25/00, A61P 25/04

(54) **TAPENTADOL FOR USE IN TREATING PAIN ASSOCIATED WITH TRIGEMINAL NEURALGIA**
TAPENTADOL ZUR BEHANDLUNG VON SCHMERZEN IM ZUSAMMENHANG MIT TRIGEMINALER NEURALGIE
TAPENTADOL POUR LE TRAITEMENT DES DOULEURS ASSOCIÉES À LA NEURALGIE DU TRIJUMEAU

(30) Priority: 05.04.2011 EP 11002810
(43) Date of publication of application: 12.02.2020
(62) Divisional of application: 12718065.1
(73) Proprietor: Grünenthal GmbH, 52078 Aachen (DE)
(72) Inventor: CHRISTOPH, Thomas, 52080 Aachen (DE); DE VRY, Jean, 2200 Herentals (BE); TZSCHENTKE, Thomas, 52080 Aachen (DE); BLOMS-FUNKE, Petra, 52146 Würselen (DE); SCHIENE, Klaus, 41363 Jüchen (DE); HAMON, Michel, 75014 Paris (FR)
(74) Representative: Kutzenberger Wolff & Partner

(56) References cited:
- WO-A2-2007/025286
- DE-A1- 102007 012 165
- US-A1- 2010 227 921
- TZSCHENTKE T M ET AL: "Tapentadol: mit zwei Mechanismen in einem Molekül wirksam gegen nozizeptive und neuropathische Schmerzen. Präklinischer Überblick = Tapentadol: with two mechanisms of action in one molecule effective against nociceptive and neuropathic pain. Preclinical overview", DER SCHMERZ ; ORGAN DER DEUTSCHEN GESELLSCHAFT ZUM STUDIUM DES SCHMERZES, DER ÖSTERREICHISCHEN SCHMERZGESELLSCHAFT UND DER DEUTSCHEN INTERDISZIPLINÄREN VEREINIGUNG FÜR SCHMERZTHERAPIE, SPRINGER, BERLIN, DE, vol. 25, no. 1, 1 February 2011 (2011-02-01), pages 19 - 25, XP009149538, ISSN: 1432-2129, [retrieved on 20110123], DOI: 10.1007/S00482-010-1004-1
- SCHRODER W ET AL: "Differential contribution of opioid and noradrenergic mechanisms of tapentadol in rat models of nociceptive and neuropathic pain", EUROPEAN JOURNAL OF PAIN, SAUNDERS, LONDON, GB, vol. 14, no. 8, 1 September 2010 (2010-09-01), pages 814 - 821, XP027224271, ISSN: 1090-3801, [retrieved on 20100611]
- CHRISTOPH T ET AL: "711 ANTI-ALLODYNIC ACTIVITY OF TAPENTADOL IN A RAT MODEL OF NEUROPATHIC PAIN DEPENDS ON OPIOID AND NORADRENERGIC, BUT NOT SEROTONERGIC, MECHANISMS", EUROPEAN JOURNAL OF PAIN, SAUNDERS, LONDON, GB, vol. 13, 1 September 2009 (2009-09-01), pages S205, XP026681308, ISSN: 1090-3801, [retrieved on 20090901], DOI: 10.1016/S1090-3801(09)60714-X
- D J WILLIAMSON ET AL: "Role of opioid receptors in neurogenic dural vasodilation and sensitization of trigeminal neurones in anaesthetized rats", BRITISH JOURNAL OF PHARMACOLOGY, vol. 133, no. 6, 1 July 2001 (2001-07-01), UK, pages 807 - 814, XP055651623, ISSN: 0007-1188, DOI: 10.1038/sj.bjp.0704136
- CHRISTENSEN DENNIS ET AL: "Combined systemic administration of the glycine/NMDA receptor antagonist, (+)-HA966 and morphine attenuates pain-related behaviour in a rat model of trigeminal neuropathic pain", PAIN, ELSEVIER SCIENCE PUBLISHERS, AMSTERDAM, NL, vol. 83, no. 3, December 1999 (1999-12-01), pages 433 - 440, XP085022862, ISSN: 0304-3959, DOI: 10.1016/S0304-3959(99)00126-8
- TZSCHENTKE T M ET AL: "Tapentadol hydrochloride. Analgesic, Mu-opioid receptor agonist, noradrenaline reuptake inhibitor", DRUGS OF THE FUTURE, PROUS SCIENCE, ES, vol. 31, no. 12, 1 December 2006 (2006-12-01), pages 1053 - 1061, XP055365206, ISSN: 0377-8282, DOI: 10.1358/DOF.2006.031.12.1047744
- ANONYMOUS: "Palexia TM retard Retardtabletten (Tapentadol)", INTERNET CITATION, 1 August 2010 (2010-08-01), pages 1 - 5, XP002643645, Retrieved from the Internet <URL:http://www.fachinfo.de/data/fi/jsearch?praep> [retrieved on 1077]
- J. ZAKRZEWSKA ET AL: "Medical management of trigeminal neuropathic pains", EXPERT OPIN. PHARMACOTHER, vol. 11, no. 8, 1 January 2010 (2010-01-01), pages 1239 - 1254, XP055767285
- DE MOULIN MD ET AL: "Pharmacological management of chronic neuropathic pain - Consensus statement and guidelines from the Canadian Pain Society", PAIN RED MANAGE, 1 January 2007 (2007-01-01), pages 13 - 21, XP055839016

## Description

The invention relates to tapentadol for use in the treatment of central neuropathic pain, namely pain associated with disorders of the trigeminal nerve, in particular for use in the treatment of pain associated with trigeminal neuralgia.

Trigeminal neuralgia (TN) or tic douloureux (also known as prosopalgia) is a neuropathic disorder of one or both of the facial trigeminal nerves. It causes episodes of intense pain in any or all of the following: the ear, eye, lips, nose, scalp, forehead, teeth or jaw on one side of the face. Trigeminal neuralgia usually develops after the age of 50, more commonly in females, although there have been cases with patients being as young as three years of age. Many ailments of the body cause pain. Generally pain is experienced when the free nerve endings constituting the pain receptors in the skin as well as in certain internal tissues are subjected to thermal, mechanical, chemical or other noxious stimuli. The pain receptors can transmit signals along afferent neurons into the central nervous system and thence to the brain.

The causes of pain can include injury, inflammation, disease, muscle spasm and the onset of a neuropathic event or syndrome. Ineffectively treated pain can be devastating to the person experiencing it by limiting function, complicating sleep, reducing mobility, and dramatically interfering with the quality of life.

Although pain arising from inflammation and muscle spasm can be initiated by mechanical or chemical stimulation of the primary sensory neuron free terminal, neuropathic pain does not require an initial stimulus to the peripheral, free nerve terminal. Neuropathic pain is a persistent or chronic pain syndrome that can result from damage to the nervous system, the peripheral nerves, the dorsal root ganglion, dorsal root, or to the central nervous system. Neuropathic pain syndromes include allodynia, various neuralgias such as post herpetic neuralgia and trigeminal neuralgia, phantom pain, and complex regional pain syndromes, such as reflex sympathetic dystrophy and causalgia.

Tragically there is no existing method for adequately, predictably and specifically treating established neuropathic pain as present treatment methods for neuropathic pain consists of merely trying to help the patient cope through psychological or occupational therapy, rather than by reducing or eliminating the pain experienced.

Central neuropathic pain is caused by damage to or dysfunction of the central nervous system (CNS), which includes the brain, brainstem, and spinal cord. It can be caused, for instance, by stroke, multiple sclerosis, tumors, epilepsy, brain or spinal cord trauma, or Parkinson's disease. Analogously, peripheral neuropathic pain occurs after damage to the peripheral nervous system (PNS), which consists of the nerves and ganglia outside of the brain and spinal cord. A central availability of an analgesic substance does not necessarily mean that said analgesic substance is effective in treating central neuropathic pain.

Peripheral and central neuropathic pain can be induced and observed in animal experiments by targeted lesions of individual nerves. The development of symptoms of neuropathic pain can subsequently be observed and quantified by means of thermal or mechanical allodynia.

A possible animal model of peripheral neuropathic pain is the nerve lesion according to Bennett and Xie (Bennett G.J. and Xie Y.K. (1988), Pain 33, 87-107), in which the sciatic nerve is bound unilaterally with loose ligatures. In contrast, the infraorbital nerve ligature as described by Vos et al. (Vos BP, Strassman AM, Maciewicz RJ (1994), J. Neurosci. 14: 2708-2723) is a known animal model for investigating central neuropathic pain. In this rat model, injury to the infraorbital nerve (one of the three branches of the trigeminal nerve) causes typical symptoms of trigeminal neuropathic pain in the animals, such as for example mechanical and thermal hyperalgesia upon stimulation on the snoud (the vibrissal pad).

Generally, it is known that analgesics are usually not equally effective in the treatment of peripheral and central neuropathic pain. For example, many opioids such as morphine may be effectively used to control peripheral neuropathic pain but only exhibit a modest effect in the treatment of central neuropathic pain syndromes. In addition, recent studies have suggested that the physiopharmacological characteristics of neuropathic pain caused by lesions of the trigeminal complex do not match completely those induced by nerve lesions in the extra-cephalic territories (Kayser et al. (2002), Br. J. Pharmacol. 137: 1287-1297; Kayser et al. (2010), Neuropharmacology, 58: 474-487; Latremoliere et al. (2008), J. Neurosci. 28: 8489-8501). In this context, it has further been shown that morphine at low doses, tetrodotoxin and 5-HT₇ receptor agonists significantly attenuate mechanical allodynia generated by chronic constriction injury to the sciatic nerve (CCI-SN) but are ineffective against that generated by chronic constriction injury to the infraorbital nerve (CCI-ION).

Consequently, there is a requirement for alternative pharmacotherapeutic methods for the treatment of central neuropathic pain, and in particular for the treatment of pain associated with disorders of the trigeminal nerve, especially for the treatment of pain associated with trigeminal neuralgia, characterized by effective pain control and a reduced side-effects profile.

The trigeminal nerve (the fifth cranial nerve) is responsible for sensation in the face. Sensory information from the face and body is processed by parallel pathways in the central nervous system. The fifth nerve is primarily a sensory nerve, but it also has certain motor functions (biting, chewing, and swallowing).

It was an object of the invention to provide medicaments for the treatment of central neuropathic pain, which medicaments have advantages over conventional drugs such as analgesics and, in particular, opioids. In particular, it was an object to find compounds that are effective in pain control related to disorders of the trigeminal nerve, in particular trigeminal neuralgia, and have advantages over the prior art.

This object is achieved by the subject matter of the claims.
Figure 1 shows the anti-hyperalgesic effects of acute treatment with tapentadol in rats with unilateral chronic constriction injury to the sciatic nerve (CCI-SN rats). Pressure threshold values to trigger hindpaw withdrawal (A) or vocalization (B) were determined using the Randall-Selitto test.
Figure 2 shows the anti-allodynic effects of acute (A) or subchronic (B) treatment with tapentadol in CCI-SN rats. Pressure threshold values were determined using the Frey filaments' test.
Figure 3 shows the anti-allodynic effects of acute (A) or subchronic (B) treatment with tapentadol in rats with unilateral chronic constriction injury to the infraorbital nerve (CCI-ION rats).
Figure 4 shows the effects of subchronic treatment with tapentadol on the levels of mRNA encoding ATF3, IL-6, BDNF or iNOS in ganglia (A) and central tissues (B) CCI-SN and sham-operated rats.
Figure 5 shows the effects of subchronic treatment with tapentadol on the levels of mRNA encoding ATF3, IL-6, BDNF or iNOS in ganglia (A) and central tissues (B) in CCI-ION and sham-operated rats.
Figure 6 shows the levels of mRNA encoding BDNF in ganglia and central tissues one (D1) and 20 (D20) days after CCI-SN, CCI-ION or sham operation.
Figure 7 shows the induction of mechanical allodynia by an intrathecal injection of BDNF in healthy rats (A) and the effect of acute treatment with tapentadol on the thus BDNF-induced allodynia (B). Pressure threshold values were determined using the Frey filaments' test.
Figure 8 shows the effects of tapentadol compared to reboxetine on mechanical allodynia induced by either intrathecal administration of BDNF (A) or CCI-SN surgery (B) in rats.
Figure 9 shows the dose-dependent anti-allodynic effect of morphine in CCI-SN rats. Pressure threshold values were determined using the Frey filaments' test.
Figure 10 shows the supra-additive anti-allodynic effects of reboxetine and morphine at low dose in CCI-SN rats. (A): Pressure threshold values were determined using the Frey filaments' test (A) and AUC values were calculated from the respective time-course curves (B).
Figure 11 shows the anti-allodynic effects of acute treatment with reboxetine and morphine, alone or combined, in CCI-SN rats. Pressure threshold values were determined using the Frey filaments' test (A) and AUC values were calculated from the respective time-course curves (B).
Figure 12 shows the supra-additive anti-allodynic effects of reboxetine and morphine in CCI-ION rats. Pressure threshold values were determined using the Frey filaments' test (A) and AUC values were calculated from the respective time-course curves (B).

The invention relates to tapentadol for use in the treatment of central neuropathic pain, associated with disorders of the trigeminal nerve, in particular for use in the treatment of pain associated with trigeminal neuralgia.

While tapentadol has been analyzed in rodent models of mono- and poly-neuropathic pain with behavioral read-outs suggesting strong analgesic potency in peripheral neuropathic pain (cf. WO 2008/110323), there is lack of knowledge on the efficacy of tapentadol on the treatment of central neuropathic pain, and, in particular, on treating pain associated with disorders of the trigeminal nerve, especially for the treatment of pain associated with trigeminal neuralgia.

The analgesic efficacy of tapentadol in the treatment of neuropathic pain is further known from DE 10 2007 012 165 A1; Lange et al., Osteoarthritis and Cartilage 18, Supplement 2 (2010), S147-S148; Tzschentke et al., Drugs of the Future 2006, 31(12): 1053-1061; Tzschentke et al., Der Schmerz 2011, 25 (1): 19-25; Schröder et al., Eur. J. Pain 2010, 14: 814-821; and from Christoph et al., Eur. J. Pain 2009, 13: S205 and from WO2007/025286.

It was surprisingly found that tapentadol combines excellent efficacy for the treatment of central neuropathic pain, in particular pain due to disorders of the trigeminal nerve,, with displaying a reduced side effect spectrum. Further, it has surprisingly been found that tapentadol is even more effective in reducing neuropathy-evoked mechanical allodynia in cephalic than in extra-cephalic territories.

Tapentadol, i.e. (-)-(1R,2R)-3-(3-dimethylamino-1-ethyl-2-methyl-propyl)-phenol (CAS no. 175591-23-8), is a synthetic, centrally acting analgesic which is effective in the treatment of moderate to severe, acute or chronic pain.

Tapentadol exhibits a dual mechanism of action, on the one hand as a µ-opioid receptor agonist and on the other as a noradrenaline transporter inhibitor. In humans, the affinity of tapentadol to the recombinantly produced µ-opioid receptor is 18-times less than that of morphine. However, clinical studies have shown the pain-alleviating action of tapentadol to be only two to three times less than that of morphine. The only slightly reduced analgesic efficacy with a simultaneously 18-times reduced affinity to the recombinant µ-opioid receptor indicates that the noradrenaline transporter inhibiting property of tapentadol also contributes to its analgesic efficacy. Consequently, it may be assumed that tapentadol has a similar analgesic efficacy to that of pure µ-opioid receptor agonists but has fewer of the side effects associated with the µ-opioid receptor. Further, due to its dual mechanism of action, it might exhibit analgesic efficacy in the treatment of pain related to disorders and/or diseases where pure µ-opioid receptor agonists only exhibit modest efficacy or even completely fail. The compound can be used in the form of its free base or as a salt or solvate. The production of the free base is known for example from EP-A 693 475.

For the purposes of the description, "tapentadol" includes (-)-(1R,2R)-3-(3-dimethylamino-1-ethyl-2-methyl-propyl)-phenol and the physiologically acceptable salts and solvates thereof, particularly the hydrochloride. Preferably, tapentadol is not provide in form of its prodrugs such as carbamates with amino acids or peptides.

Suitable physiologically acceptable salts include salts of inorganic acids, such as e.g. hydrogen chloride, hydrogen bromide and sulfuric acid, and salts of organic acids, such as methanesulfonic acid, fumaric acid, maleic acid, acetic acid, oxalic acid, succinic acid, malic acid, tartaric acid, mandelic acid, lactic acid, citric acid, glutaminic acid, acetylsalicylic acid, nicotinic acid, aminobenzoic acid, α-lipoic acid, hippuric acid and aspartic acid.

The most preferred salt is hydrochloride.

Tapentadol can also be present as a mixture of salts of the above-mentioned organic and inorganic acids in any desired ratio.

In a preferred embodiment, tapentadol is present in solid form. Liquid or pasty medicinal forms are also possible.

According to the present invention, tapentadol is formulated for oral administration. However, pharmaceutical forms that are adapted for other administration routes are also possible, for example buccal, sublingual, transmucosal, rectal, intralumbar, intraperitoneal, transdermal, intravenous, intramuscular, intragluteal, intracutaneous and subcutaneous administration.

Depending upon the formulation, the tapentadol preparation preferably contains suitable additives and/or excipients. Suitable additives and/or excipients for the purpose of the invention are all substances for achieving galenic formulations known to the person skilled in the art from the prior art. The selection of these excipients and the amounts to use depend upon how the medicinal product is to be administered, i.e. orally, intravenously, intraperitoneally, intradermally, intramusuclarly, intranasally, buccally or topically.

Suitable for oral administration are preparations in the form of tablets, chewable tablets, dragees, capsules, granules, drops, juices or syrups; suitable for parenteral, topical and inhalative administration are solutions, suspensions, easily reconstituted dry preparations and sprays.

Examples of excipients and additives for oral administration forms are disintegrants, lubricants, binders, fillers, mould release agents, optionally solvents, flavourings, sugar, in particular carriers, diluents, colorants, antioxidants, etc.

Excipients can be for example: water, ethanol, 2-propanol, glycerin, ethylene glycol, propylene glycol, polyethylene glycol, polypropylene glycol, glucose, fructose, lactose, sucrose, dextrose, molasses, starch, modified starch, gelatin, sorbitol, inositol, mannitol, microcrystalline cellulose, methyl cellulose, carboxymethylcellulose, cellulose acetate, shellac, cetyl alcohol, polyvinylpyrrolidone, paraffins, waxes, natural and synthetic rubbers, acacia gum, alginates, dextran, saturated and unsaturated fatty acids, stearic acid, magnesium stearate, zinc stearate, glyceryl stearate, sodium lauryl sulfate, edible oils, sesame oil, coconut oil, groundnut oil, soybean oil, lecithin, sodium lactate, polyoxyethylene and propylene fatty acid ester, sorbitan fatty acid esters, sorbic acid, benzoic acid, citric acid, ascorbic acid, tannic acid, sodium chloride, potassium chloride, magnesium chloride, calcium chloride, magnesium oxide, zinc oxide, silicon dioxide, titanium oxide, titanium dioxide, magnesium sulfate, zinc sulfate, calcium sulfate, potash, calcium phosphate, dicalcium phosphate, potassium bromide, potassium iodide, talc kaolin, pectin, crospovidone, agar and bentonite.

The production of this tapentadol preparation is performed with the aid of means, devices, methods and processes which are well known in the prior art of pharmaceutical formulation, such as those described for example in "Remington's Pharmaceutical Sciences", ed AR Gennaro, 17th edition, Mack Publishing Company, Easton, pa. (1985), in particular in Part 8, Chapters 76 to 93.

For example, for a solid formulation, such as a tablet, the tapentadol can be granulated with a pharmaceutical carrier, e.g. conventional tablet ingredients, such as maize starch, lactose, sucrose, sorbitol, talc, magnesium stearate, dicalcium phosphate or physiologically acceptable rubbers, and pharmaceutical diluents, such as water, for example, to form a solid composition containing the tapentadol in a homogeneous distribution. Here, a homogeneous distribution should be understood as meaning that the tapentadol is distributed uniformly throughout the entire composition so that this can be easily divided into equally effective single dose forms, such as tablets, capsules, dragees. The solid composition is then divided into single dose forms. The tablets or pills can also be coated or compounded in some other way in order to produce a dosage form with delayed release. Suitable coating means are inter alia polymers acids and mixtures of polymeric acids with materials such as shellac, for example, cetyl alcohol and/or cellulose acetate.

In a preferred embodiment of the present invention tapentadol is present in immediate release form.

In another preferred embodiment of the present invention tapentadol is present in controlledrelease form.

The term controlled release as used herein refers to any type of release other than immediate release such as delayed release, prolonged release, sustained release, slow release, extended release and the like. These terms are well known to any person skilled in the art as are the means, devices, methods and processes for obtaining such type of release.

Controlled release of tapentadol is possible from formulations for oral, rectal or percutaneous administration. Preferably, the tapentadol is formulated for once-daily administration, for twice-daily administration *(bid)* or for thrice-daily administration, with twice-daily administration *(bid)* being particularly preferred.

The controlled release of tapentadol can, for example, be achieved by retardation by means of a matrix, a coating or release systems with an osmotic action (see e.g. US-A-2005-58706). The invention also relates to a pharmaceutical dosage form comprising tapentadol for use in the treatment of central neuropathic pain, associated with disorders of the trigeminal nerve, in particular for use in the treatment of pain associated with trigeminal neuralgia.

Preferably, the pharmaceutical dosage form is adapted for once-daily administration, for twice-daily administration *(bid)* or for thrice-daily administration, with twice-daily administration *(bid)* being particularly preferred.

The pharmaceutical dosage form may contain one or more further drugs besides tapentadol. Preferably, however, the tapentadol formulation contains tapentadol as the only drug.

In a preferred embodiment, the pharmaceutical dosage form contains a vitamin, preferably vitamin B complex.

The amounts of tapentadol to be administered to patients vary depending upon the weight of the patient, the method of administration and the severity of the disease and/or pain. Tapentadol may be administered in amounts up to its maximum daily dosage, which is known to those skilled in the art. In a preferred embodiment, the pharmaceutical dosage form contains tapentadol in an amount of 10 to 300 mg, more preferably 20 to 290 mg, even more preferably 30 to 280 mg, most preferably 40 to 260 mg, as an equivalent dose based on the free base.

In a preferred embodiment, the mean serum concentration of tapentadol, following twice-daily administration of the pharmaceutical dosage form over a period of at least three days, more preferably at least four days and in particular at least five days, is on average at least 5.0 ng/ml, at least 10 ng/ml, at least 15 ng/ml or at least 20 ng/ml, more preferably at least 25 ng/ml or at least 30 ng/ml, even more preferably at least 35 ng/ml or at least 40 ng/ml, most preferably at least 45 ng/ml or at least 50 ng/ml and in particular at least 55 ng/ml or at least 60 ng/ml. This means that tapentadol is administered over a period of at least three days twice daily and then, preferably 2 h after the administration, the serum concentration is measured. The authoritative numerical value is then obtained as the mean value for all the patients investigated.

In a preferred embodiment, the mean serum concentration of tapentadol in at the most 50% of the patient population, which preferably comprises at least 100 patients, more preferably in at the most 40%, even more preferably in at the most 30%, most preferably in at the most 20% and in particular in at the most 10% of the patient population, following twice-daily administration over a period of at least three days, more preferably at least four days and in particular at least five days, is on average less than 5.0 ng/ml, preferably less than 7.5 ng/ml, even more preferably less than 10 ng/ml, most preferably less than 15 ng/ml and in particular less than 20 ng/ml.

In a preferred embodiment, the mean serum concentration of tapentadol in at the most 50% of the patient population, comprising preferably at least 100 patients, more preferably in at the most 40%, even more preferably in at the most 30%, most preferably in at the most 20% and in particular in at the most 10% of the patient population, following twice-daily administration over a period of at least three days, more preferably at least four days and in particular at least five days, is on average more than 300 ng/ml, more preferably more than 275 ng/ml, even more preferably more than 250 ng/ml, most preferably more than 225 ng/ml and in particular more than 200 ng/ml.

Preferably, the mean serum concentration of tapentadol in at least 50% or 55% of the patient population, which preferably comprises at least 100 patients, more preferably in at least 60% or 65%, even more preferably in at least 70% or 75%, most preferably in at least 80% or 85% and in particular in at least 90% or 95% of the patient population, following twice-daily administration over a period of at least three days, more preferably at least four days and in particular at least five days, is on average in the range of from 1.0 ng/ml to 500 ng/ml, more preferably in the range of from 2.0 ng/ml to 450 ng/ml, even more preferably in the range of from 3.0 ng/ml to 400 ng/ml, most preferably in the range of from 4.0 ng/ml to 350 ng/ml and in particular in the range of from 5.0 ng/ml to 300 ng/ml.

In a preferred embodiment, the percentage standard deviation (coefficient of variation) of the mean serum concentration of tapentadol, preferably in a patient population of 100 patients, following twice-daily administration of the pharmaceutical dosage form over a period of at least three days, more preferably at least four days and in particular at least five days, is at the most ± 90%, more preferably at the most ± 70%, even more preferably at the most ± 50%, at the most ± 45% or at the most ± 40%, most preferably at the most ± 35%, at the most ± 30% or at the most ± 25% and in particular at the most ± 20%, at the most ± 15% or at the most ± 10%.

Preferably, the serum concentrations are average values, produced from measurements on a patient population of preferably at least 10, more preferably at least 25, even more preferably at least 50, even more preferably at least 75, most preferably at least 100 and in particular at least 250 patients. A person skilled in the art knows how to determine the serum concentrations of tapentadol. In this context, reference is made, for example, to TM Tschentke et al, Drugs of the Future, 2006, 31 (12), 1053.

In a preferred embodiment the tapentadol or the pharmaceutical dosage form, respectively,
- is present in a solid and/or pressed and/or film-coated medicinal form; and/or
- is present in a controlled release form; and/or
- contains tapentadol in a amount of 0.001 to 99.999 % by weight, more preferably 0.1 to 99.9 % by weight, even more preferably 1.0 to 99.0 % by weight, even more preferably 2.5 to 80 % by weight, most preferably 5.0 to 50 % by weight and in particular 7.5 to 40 % by weight, based on the total weight of the pharmaceutical dosage form; and/or
- contains a physiologically acceptable carrier and/or physiologically acceptable excipients; and/or
- has a total mass in the range of from 25 to 2,000 mg, more preferably 50 to 1,800 mg, even more preferably 60 to 1,600 mg, even more preferably 70 to 1,400 mg, most preferably 80 to 1,200 mg and in particular 100 to 1,000 mg, and/or
- is selected from the group consisting of tablets, capsules, pellets and granules.

The pharmaceutical dosage form can be provided as a simple tablet and as a coated tablet (e.g. as a film-coated tablet or dragee). The tablets are usually round and biconvex, but oblong shapes are also possible. Granules, spheroids, pellets or microcapsules, which are used to fill sachets or capsules or pressed into disintegrating tablets, are also possible. Pharmaceutical dosage forms containing at least 0.001 to 99.999 % tapentadol, in particular low, active doses, are preferred in order to avoid side effects. The pharmaceutical dosage form contains preferably 0.01 % by weight to 99.99 % by weight tapentadol, more preferably 0.1 to 90 % by weight, even more preferably 0.5 to 80 % by weight, most preferably 1.0 to 50 % by weight and in particular 5.0 to 20 % by weight. To avoid side effects, it may be advantageous at the start of the treatment to increase the amount of tapentadol to be administered gradually (titration) to allow the body to become accustomed to the active substance slowly. Preferably, tapentadol is first administered in a dose which is below the analgesically active dose.

Particularly preferably, the pharmaceutical dosage form is an oral administration form, which is formulated for twice-daily administration and contains tapentadol in an amount of 20 to 260 mg as an equivalent dose based on the free base.

In one of its embodiments, the present invention relates to tapentadol for use in the treatment of pain associated with disorders of the trigeminal nerve, in particular for the treatment of pain associated with trigeminal neuralgia.

Preferably, the pain is in the ear, eye, lips, nose, scalp, forehead, teeth or jaw on one side or both sides of the face.

The tapentadol is for use in the treatment of pain associated with disorders of the trigeminal nerve.

Preferably, the disorders of the trigeminal nerve are selected from the group consisting of trigeminal neuralgia and atypical facial pain; in particular the disorder of the trigeminal nerve is trigeminal neuralgia.

Preferably, the tapentadol is for use in the treatment of pain associated with disorders of the trigeminal nerve in any or all of the following: the ear, eye, lips, nose, scalp, forehead, facial skin, teeth or jaw on one side or on both sides of the face.

Preferably, the disorders of the trigeminal nerve are as defined by ICD-10 (International Statistical Classification of Diseases and Related Health Problems, WHO edition, preferably 2007 version), i.e. the disorders of the trigeminal nerve are selected from trigeminal neuralgia [G50.0], atypical facial pain [G50.1], other disorders of the trigeminal nerve [G50.8] and unspecified disorders of the trigeminal nerve [G50.9]. The references in brackets refer to the ICD-10 nomenclature.

If the disorder of the trigeminal nerve is trigeminal neuralgia [G50.0], it preferably includes disorders of the 5th cranial nerve. Furthermore, if the disorder of the trigeminal nerve is trigeminal neuralgia [G50.0], this is preferably selected from the group consisting of the syndrome of paroxysmal facial pain and tic douloureux.

Preferably, the pain is moderate to strong (severe).

Even if the tapentadol according to the invention exhibits few side effects only, it may be advantageous, for example, in order to avoid certain types of dependency to use morphine antagonists, in particular naloxone, naltrexone and/or levallorphan, in addition to tapentadol.

The present disclosure also relates to a kit containing the pharmaceutical dosage form according to the invention.

The kit according to the present disclosure is preferably designed for in each case once daily, twice daily or three times daily administration of the pharmaceutical dosage forms contained therein.

The following examples serve for a further explanation of the invention but should not be construed as restrictive.

### Examples:

### Effects of tapentadol on allodya/hyperalgesia in rats with ligatures of the infraorbital nerve versus the sciatic nerve

The sciatic nerve ligature as described by Bennett and Xie (Bennett G.J. and Xie Y.K. (1988), Pain 33, 87-107) serves as a model of peripheral mono-neuropathic pain. In contrast, the infraorbital nerve ligature as described by Vos et al. (Vos BP, Strassman AM, Maciewicz RJ (1994), J. Neurosci. 14: 2708-2723) serves as a model of central (mono-)neuropathic pain representing aspects of trigeminal neuralgia.

In the following experiments the analgesic potential of tapentadol in both pain models was analyzed and compared to that of reboxetine, morphine and to a combination of these two drugs. Reboxetine is known to inhibit the reuptake of norepinephrine (NA), whereas morphine serves as an example for a potent µ-opioid receptor agonist.

### I. Animals

Male Sprague-Dawley rats (Breeding center: Charles River Laboratories, L'Arbresle, France), weighing 150-200 g at arrival, are used. Animals are maintained under controlled conditions (22 ± V C, 60 % relative humidity, 12 h/12 h light/dark cycle, food and water ad libitum) starting from reception in the laboratory, for at least 1 week before any treatment/intervention and thereafter, until euthanasia.

### II. Surgical procedures (Induction of CCI-SN or CCI-ION)

### a) Chronic constriction injury to the sciatic nerve (CCI-SN)

Rats are anaesthetized with sodium pentobarbital (50 mg/kg i.p.). Unilateral CCI-SN is performed under direct visual control using a Zeiss microscope (10-25x) essentially as described by Bennett and Xie (G.J. Bennett et al., Pain, 33 (1988) 87-107).

### b) Chronic constriction injury to the infraorbital nerve (CCI-ION)

Rats are anaesthetized with sodium pentobarbital (50 mg/kg i.p.). Unilateral CCI-ION is performed under direct visual control using a Zeiss microscope (10-25x) essentially as described by Vos et al (Vos BP, Strassman AM, Maciewicz RJ (1994), J. Neurosci. 14: 2708-2723). Briefly, the head is fixed in a Horsley-Clarke stereotaxic frame and a midline scalp incision is made, exposing skull and nasal bone. The edge of the orbit, formed by the maxillary, frontal, lacrimal, and zygomatic bones, is dissected free. The orbital contents are gently deflected to give access to the infraorbital nerve which is dissected free at its most rostral extent in the orbital cavity, just caudal to the infraorbital foramen. Only 5 mm of the nerve can be freed (Vos et al.), providing the space for placement of two chromic catgut (5-0) ligations tied loosely (with about 2 mm spacing) around it. To obtain the desired degree of constriction, the criterion formulated by Bennett and Xie (Bennett GJ, Xie YK (1988), Pain 33: 87-107) is used: the ligations reduce the diameter of the nerve by a just noticeable amount and retard, but do not interrupt the epineural circulation. Finally, scalp incision is closed using silk sutures (4-0). In sham-operated rats, the ION is exposed using the Saure procedure, but is not ligated.

### III. Pharmacological treatments and behavioral tests (general procedures)

Pharmacological treatments are started 14 days after surgery, when allodynia/ hyperalgesia reaches a plateau in CCI rats (Latremoliere A, Mauborgne A, Masson J, Bourgoin S, Kayser V, Hamon M, Pohl M. (2008), J. Neurosci. 28: 8489-8501).

All behavioral assessments were conducted between 09:00 and 17:00 h in a quiet room. Rats are placed individually in small (35 x 20 x 15 cm) plastic cages for a 2 h habituation period.

### a) Randall-Selitto test in CCI-ION rats

Pain is produced by applying increasing pressure (0-450 g/mm²) with a punch (0.2 mm tip diameter) on the rat's hind paw. The measured value to be determined is the pressure at which either a hindpaw withdrawal response or a vocalisation reaction of the animal occurs.

### b) von Frey filament test in CCI-ION rats

Mechanical sensitivity is determined with a graded series of eleven von Frey filaments (Bioseb, Bordeaux, France). The filaments produce a bending force of 0.07, 0.16, 0.40, 0.60, 1.00, 2.00, 4.00, 6.00, 8.00, 10.00 and 12.00 g, respectively. The stimuli are applied within the ION territory (vibrissae pad) three times at the nerve-injured side and then at the contralateral side for a total of 6 applications of each filament per rat, always beginning with the filament producing the lowest force. The von Frey filaments are applied at least 3 seconds after the rats returned to their initial resting state. For each session, the complete series of von Frey filaments is tested in increasing force order. Behavioral nociceptive response consists of either
(1) a brisk withdrawal reaction: the rat pulls briskly backward; or
(2) an escape/attack: the rat avoids further contact with the filament either passively by moving its body away from the stimulating object to assume a crouching position against cage wall, sometimes with the head buried under the body, or actively by attacking the stimulating object, making biting and grabbing movements; or
(3) asymmetric face grooming: the rat displays an uninterrupted series of at least 3 facewash strokes directed to the stimulated facial area, often preceded by the brisk withdrawal reaction.

The latter responses represent the highest scores in the rank-ordered response scoring system initially described by Vos et al. The minimal force filament causing at least one among these responses (to at least 2 out of the 3 an each side) allows determination of the mechanical response threshold. The 12.00 g filament is the cut-off threshold (no tissue-injury occurs at this pressing force).

### c) von Frey filament test in CCI-SN rats

Mechanical sensitivity is determined with a graded series of von Frey filaments (Bioseb, Bordeaux, France; bending force: 0.07 - 60.0 g). The stimuli are applied within the SN territory (midplantar surface of the left hindpaw) three times at the nerve-injured side (ipsilateral, left) and then at the contralateral side for a total of 6 applications of each filament per rat, always beginning with the filament producing the lowest force. The von Frey filaments are applied at least 3 seconds after the rats returned to their initial resting state. For each session, the complete series of von Frey filaments is tested in increasing force order. The minimal force filament causing a hindpaw withdrawal response allows determination of the mechanical response threshold. The 60 g filament is the cut-off threshold.

### V. Statistical Analyses

Data are expressed as means ± S.E.M. Repeated measures' analysis of variance (ANOVA) or one-way ANOVA when appropriate is conducted to compare the time effect and group differences in the study of drug's effects an the behavioral responses. When ANOVA indicates a significant difference between groups, data are further analyzed using a post hoc Fisher's protected least significant difference (PLSD) test. Areas under the time-course curves (AUC) are calculated using the trapezoidal rule. Differences between AUC values in two groups are evaluated using Student' t-test. The significance level is set at P < 0.05.

### Comparative Example 1

### Effects of acute or subchronic treatment with tapentadol in CCI-SN rats

### Randall-Selitto test

Tapentadol (10 mg/kg i.p.) or its vehicle (0.9% NaCl i. p.) was injected in CCI-SN rats acutely 14 days after surgery. Pressure threshold values to trigger hindpaw withdrawal (A) or vocalization (B) were determined using the Randall-Selitto test at various times after acute i.p. administration (Time = 0) of Tapentadol or saline.

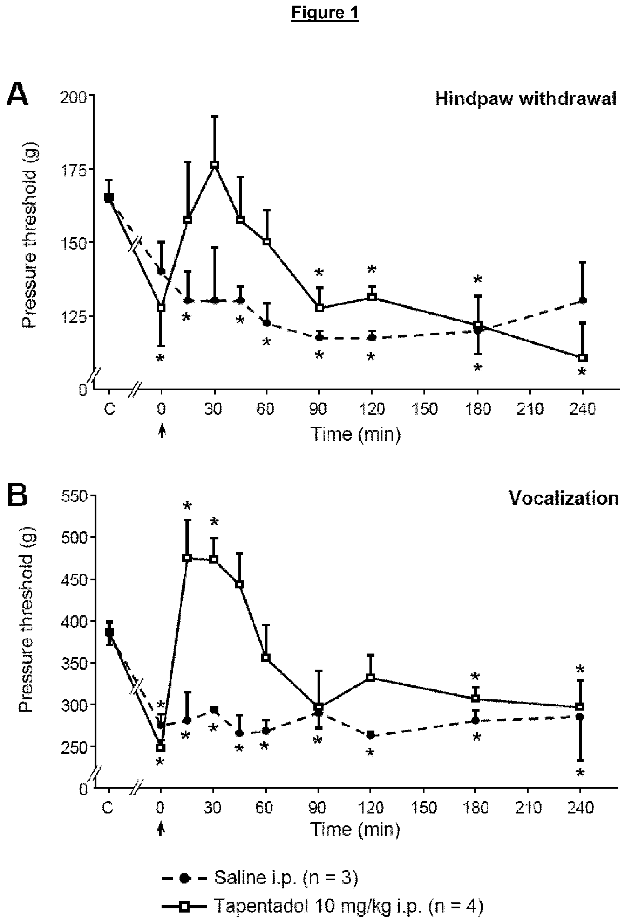

The results are summarized and depicted in Figure 1 (A and B). Each point is the mean ± S.E.M. of 3-4 independent determinations. * P < 0.05 compared to pressure threshold values in the same rats before surgery (C on abscissa); Dunnett's test.

It becomes evident from Figure 1 that two weeks after unilateral ligation of the sciatic nerve, pressure threshold values to evoke withdrawal of hindpaw ipsilateral to CCI-SN (Fig. 1A) and vocalization (Fig. 1B) were significantly decreased. At this time, acute i.p. administration of saline did not significantly affect CCI-SN-induced decreases in both pressure threshold values (Fig. 1A,B). In contrast, tapentadol, at the dose of 10 mg/kg i.p., produced a rapid increase in these values, which lasted for at least 60 min after the drug administration. Indeed, for the first hour after tapentadol treatment, pressure threshold values to cause hindpaw withdrawal did not significantly differ from those determined in intact healthy rats, before surgery for nerve ligations (Fig. 1A). Regarding vocalization, pressure threshold values to trigger this response were even slightly higher (+20%) for the first 45 min after Tapentadol administration in CCI-SN rats than in untreated healthy rats (Fig. 1B), suggesting the occurrence of an analgesic effect in addition to reversal of CCI-SN-induced hyperalgesia.

### Von Frey filament test

For studying the effects of acute treatment with tapentadol in CCI-SN rats, tapentadol (1, 3 and 10 mg/kg i.p.) or its vehicle (0.9% NaCl i. p.) was injected acutely 14 days after surgery. Sham-operated rats were treated in parallel. Pressure threshold values to trigger nocifensive responses to von Frey filaments application onto the plantar surface of ipsilateral hindpaw were determined at various times after acute injection of tapentadol or saline. Cut-off was fixed at 60 g pressure.

For studying the effects of subchronic treatment with tapentadol in CCI-SN rats, starting on day 16 (Time = D) after surgery, they received i.p. injections of tapentadol (10 mg/kg, twice daily, at 10:00 AM and 6:00 PM) or saline (at the same day times as the drug) for four days. On the following day (day 20 after surgery), both tapentadol- and saline-pretreated CCI-SN rats were i.p. injected with Tapentadol (10 mg/kg ; Time = 0) and then subjected to von Frey filaments'test applied to ipsilateral hindpaw for determination of pressure threshold values at various times thereafter.

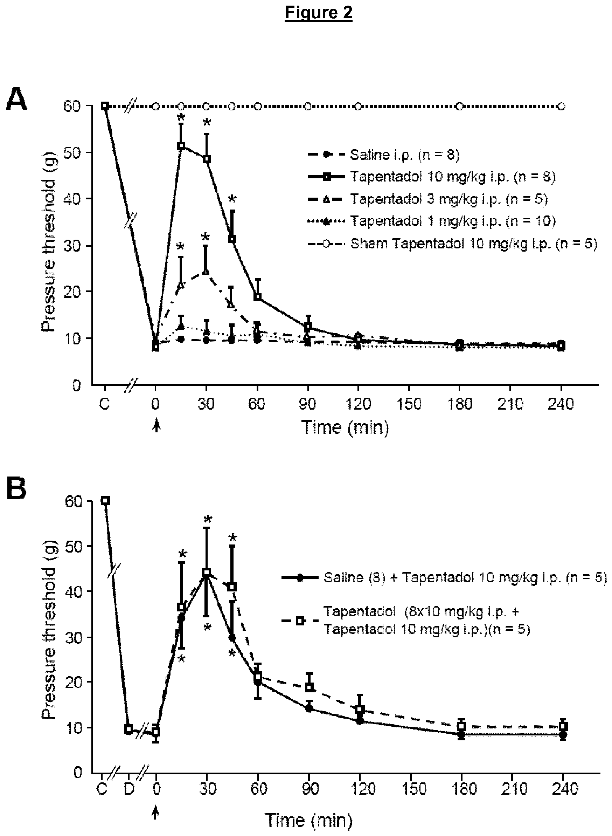

The results are summarized and depicted in Figure 2A (acute treatment) and Figure 2B (subchronic treatment). Each point is the mean ± S.E.M. of 5-10 independent determinations (as indicated in parentheses). * P < 0.05 compared to pressure threshold values determined in CCI-SN rats just prior to Tapentadol or saline injection (arrow, 0 on abscissa) ; Dunnett's test.

It becomes evident from Figure 2A, that the pressure threshold to trigger hindpaw withdrawal in response to plantar application of von Frey filaments on the ligated side was lowered by 90% compared to intact healthy rats (compare pressure values corresponding to 0 versus C on abscissa in Figure 2A). At the dose of 1 mg/kg i.p., tapentadol exerted a discrete effect only, resulting in an approximately 40% (non significant) increase in pressure threshold value for the first 15-30 min after injection (Figure 2A). In contrast, a huge effect was noted after the administration of 10 mg/kg i.p. of Tapentadol since pressure threshold values no longer differed from healthy control values (C on abscissa) in CCI-SN rats at 15-30 min after the drug administration (Figure 2A). Thereafter, the effect of Tapentadol progressively disappeared, and 90 min after the drug injection, mechanical allodynia did not significantly differ from that measured in saline-treated CCI-SN rats. At the dose of 3 mg/kg i.p., Tapentadol also increased pressure threshold values, but to a lower extent than 10 mg/kg i.p., indicating a clear dose-dependent anti-allodynic effect of the drug in the 1-10 mg/kg i.p. dose range in CCI-SN rats (Figure 2A).

The data in Figure 2B show that the last administration of tapentadol produced the same anti-allodynic effects in saline-pretreated and tapentadol-pretreated rats. As after acute treatment in the previous series of experiments (Figure 2A), Tapentadol markedly increased pressure threshold values for the first 45 min after the last injection under subchronic treatment conditions, and this effect progressively vanished following similar time course whether or not CCI-SN rats had been pretreated with the drug (Figure 2B).

### Example 2

### Effects of acute or subchronic treatment with tapentadol in CCI-ION rats

For studying the effects of acute treatment with tapentadol in CCI-ION rats, tapentadol (1 or 10 mg/kg i.p.) or its vehicle (0.9% NaCl i. p.) was injected acutely 14 days after surgery. Sham-operated rats were treated in parallel. Pressure threshold values to trigger nocifensive responses to von Frey filaments application onto the plantar surface of ipsilateral hindpaw were determined at various times after acute injection of tapentadol or saline. Cut-off was fixed at 12 g pressure.

For studying the effects of subchronic treatment with tapentadol in CCI-ION rats, starting on day 16 (Time = D) after surgery, they received i.p. injections of tapentadol (10 mg/kg, twice daily, at 10:00 AM and 6:00 PM) or saline (at the same day times as the drug) for four days. On the following day (day 20 after surgery), both tapentadol- and saline-pretreated CCI-SN rats were i.p. injected with Tapentadol (10 mg/kg ; Time = 0) and then subjected to von Frey filaments'test applied to ipsilateral hindpaw for determination of pressure threshold values at various times thereafter.

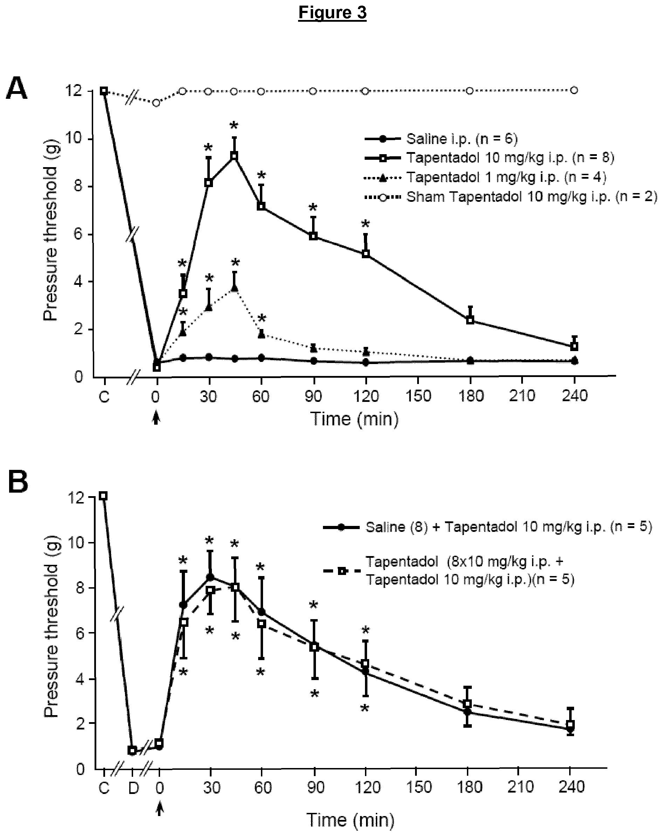

The results are summarized and depicted in Figure 3A (acute treatment) and Figure 3B (subchronic treatment). Each point is the mean ± S.E.M. of the number of independent determinations indicated in parentheses. * P < 0.05 compared to pressure threshold values determined in CCI-SN rats just prior to Tapentadol or saline injection (arrow, 0 on abscissa); Dunnett's test.

As shown in Figure 3A, two weeks after the surgery, pressure threshold value to trigger nocifensive response to the application of von Frey filaments onto vibrissal pad in CCI-ION rats was less than 5% of that determined in intact healthy rats (compare 0 to C on abscissa). At this time, acute i.p. administration of saline was ineffective, but Tapentadol at 1 mg/kg i.p. produced an up to a 6-fold increase in pressure threshold value compared to that determined in saline-treated CCI-ION rats. This increase developed progressively for the first 45 min after Tapentadol injection, then pressure threshold values returned, within the following 45 min, down to the same bottom level as that found in saline-treated CCI-ION rats (Figure 3A). At the dose of 10 mg/kg i.p., the anti-allodynic effect of Tapentadol was markedly larger in both amplitude and duration since, 30-60 min after the drug injection, pressure threshold values were up to 15-20-fold higher than those determined prior to injection. Furthermore, at this dose, the anti-allodynic effect of Tapentadol assessed through drug-induced increase in pressure threshold values remained statistically significant for more than two hours after the drug injection (Figure 3A).

It becomes evident from Figure 3B that the anti-allodynic effect of tapentadol (10 mg/kg i.p.) had the same characteristics (amplitude, duration) whether CCI-ION rats had received repeated injections of saline (twice daily, at 10:00 and 18:00) or Tapentadol (10 mg/kg i.p. at 10:00 and 18:00) for the four preceding days (Fig. 3B).

Therefore, neither in CCI-SN rats nor in those with CCI-ION, any sign of sensitization or desensitization to tapentadol under subchronic treatment conditions was detected (cf. Figures 2B and 3B).

### Comparative Example 3

### Effects of subchronic treatment with Tapentadol on the levels of mRNA encoding ATF3, IL-6, iNOS and BDNF in ganglia and central tissues in CCI-SN- rats versus respective sham-operated rats

Real-time qRT-PCR determinations were made on the 20th day after CCI-SN or sham operation. Saline or Tapentadol was administered at days 16-20 under treatment conditions according to Example 2 (subchronic treatment conditions). Rats were decapitated 4 h after the last injection on day 20, tissues were immediately dissected in the cold (0°C) and processed for mRNA extraction and quantification as described by Latrémolière et al. (J. Neurosci. 2008, 28, 8489-8501).

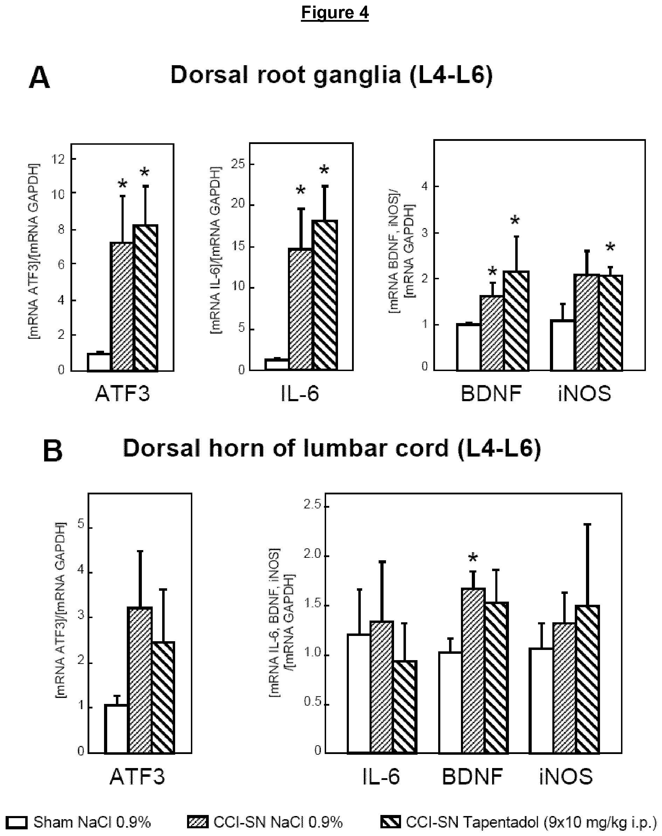

The results are summarized in Figure 4.

mRNA levels are expressed with reference to transcrip encoding the reporter gene GaPDH (glyceraldehyde 3-phosphate dehydrogenase). Each bar is the mean ± S.E.M. of 4-6 independent determinations.

* P < 0.05 compared to respective values in sham-operated rats ; Fisher's protected least significant difference *post hoc* test.

Marked overexpressions of ATF3 mRNA (by about seven-fold) and IL-6 mRNA (by about 15-fold) were found in ipsilateral (to the ligated sciatic nerve) L4-L6 dorsal root ganglia (DRG) of CCI-SN rats compared to sham-operated animals. In addition, BDNF mRNA levels and iNOS mRNA levels were also higher in L4-L6 DRG of CCI-SN compared to sham rats, but their overexpression was less than that of the previous two markers (Figure 4A).

In the ipsilateral dorsal quadrant of the lumbar enlargement of the spinal cord at L4-L6, clear-cut increases in ATF3- and BDNF-mRNA levels in CCI-SN compared to sham rats were found (Figure 4B). In contrast, the levels of mRNAs encoding IL-6 and iNOS were not significantly affected by CCI-SN (Figure 4B).

As illustrated in Figures 4A and 4B, levels of mRNA encoding ATF3, IL-6, BDNF and iNOS in both ipsilateral DRG and dorsal quadrant of the lumbar enlargement of the spinal cord were not significantly different in Tapentadol-versus saline-treated CCI-SN rats. These data suggest that, under the conditions used for subchronic treatment, Tapentadol did not interfere with the overexpression of neuroinflammatory and trophic factors caused by CCI-SN.

### Example 4

### Effects of subchronic treatment with Tapentadol on the levels of mRNA encoding ATF3, IL-6, iNOS and BDNF in ganglia and central tissues in CCI-ION- rats versus respective sham-operated rats

Real-time qRT-PCR determinations were made on the 20th day after CCI-SN or sham operation. Saline or Tapentadol was administered at days 16-20 under treatment conditions according to Example 2 (subchronic treatment conditions). Rats were decapitated 4 h after the last injection on day 20, tissues were immediately dissected in the cold (0°C) and processed for mRNA extraction and quantification as described by Latrémolière et al. (2010, Neuropharmacology, 58, 474-487).

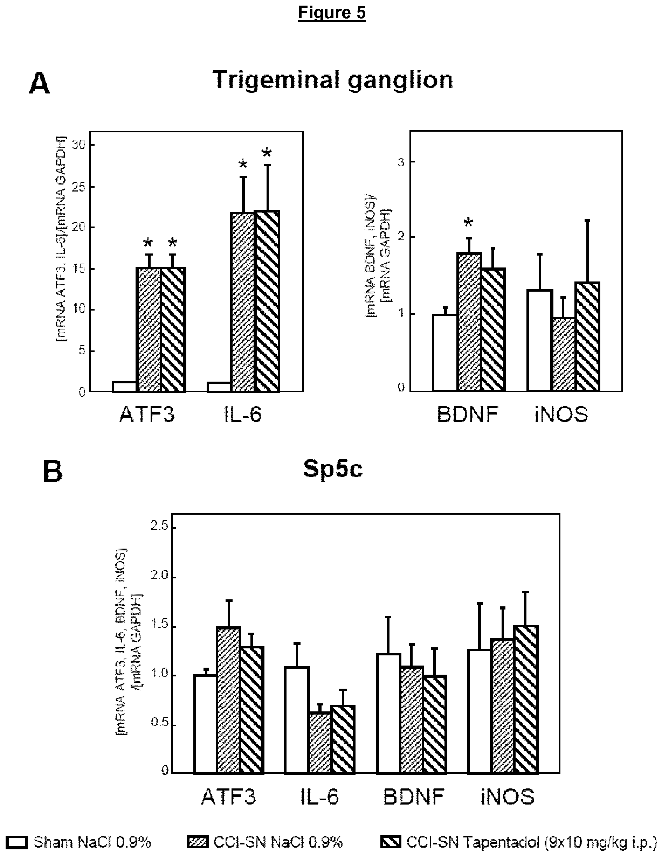

The results are summarized in Figure 5.

mRNA levels are expressed with reference to mRNA encoding the reporter gene GaPDH (glyceraldehyde 3-phosphate dehydrogenase). Each bar is the mean ± S.E.M. of 4-6 independent determinations.

* P < 0.05 compared to respective values in sham-operated rats ; Fisher's protected least significant difference *post hoc* test.

Marked overexpressions of ATF3 mRNA (by about 15-fold) and IL-6 mRNA (by more than 20-fold) were observed in the trigeminal ganglion on the lesioned side in CCI-ION- compared to sham-operated rats (Figure 5A). An apparent up-regulation of BDNF, but not iNOS, gene transcription was also observed in the ipsilateral trigeminal ganglion of ION ligated- versus sham-rats (Figure 5A).

In the caudal portion of the ipsilateral spinal nucleus of the trigeminal nerve (Sp5c), only moderate, non-significant, changes in the levels of mRNA encoding ATF3, IL-6, BDNF were noted in CCI-ION- versus paired sham-animals (Figure 5B). In particular, some tendencies to increased levels of ATF3 mRNA and decreased levels of IL-6 mRNA were found, but BDNF mRNA levels were clearly unchanged in CCI-ION- compared to sham-rats.

Interestingly, subchronic treatment with Tapentadol had no significant effects on ATF3, IL-6 , BDNF and iNOS mRNA levels in both the trigeminal ganglion and the spinal nucleus of the trigeminal nerve ipsilateral to nerve ligations in CCI-ION rats (Figures 5A and B).

The real-time qRT-PCR determinations according to Examples 3 and 4 showed significant increases in BDNF mRNA levels in ipsilateral peripheral ganglia in both CCI-SN and CCI-ION rats, but only in central tissues of CCI-SN rats (ipsilateral dorsal quadrant of the lumbar enlargement of the spinal cord) compared to sham animals. Indeed, BDNF mRNA levels were not significantly modified in the ipsilateral Sp5c of CCI-ION- versus paired sham-rats (Figure 5B).

However these real-time qRT-PCR determinations of specific mRNA levels were made only at day 20 (i.e. after a two-week recovery period after surgery followed by a 5-day treatment with saline or Tapentadol), therefore providing no information regarding the possible induction of BDNF expression also in Sp5c, but at earlier times after surgery in CCI-ION rats. Accordingly, in the next study real-time qRT-PCR mRNA determinations were performed as soon as 24 h (day 1) after surgery:

### Example 5

### Expression of BDNF mRNA in central tissues in CCI-SN compared to CCI-ION rats 24 h after surgery

According to Examples 3 and 4, real-time qRT-PCR determinations were made 24 h after surgery.

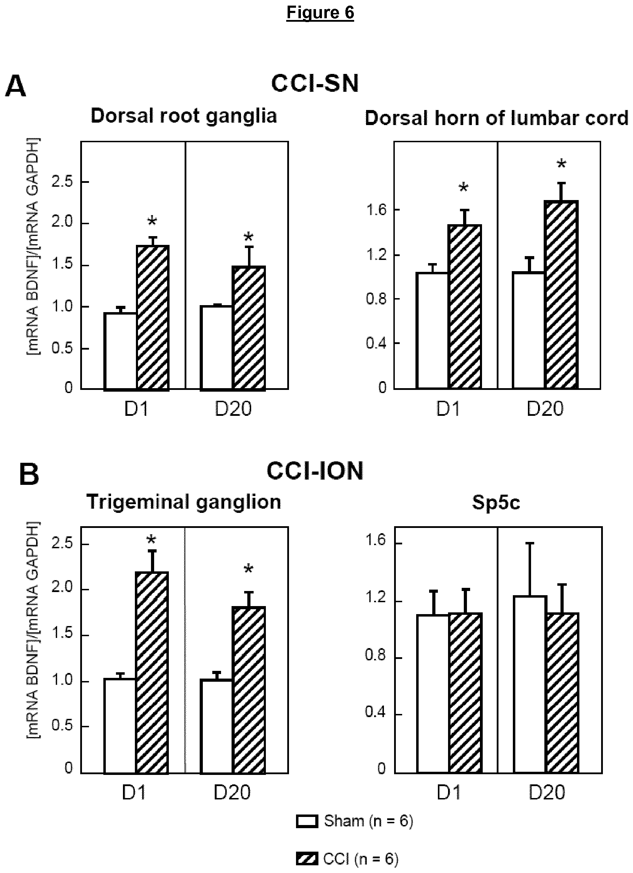

In Figure 6 the results of this study are summarized and compared to the results according to Examples 3 and 4.

It becomes evident from Figure 6A that BDNF mRNA levels were upregulated in both ipsilateral L4-L6 DRG and dorsal quadrant of the lumbar enlargement of the spinal cord already at one day after CCI-SN. Indeed, this effect was as pronounced as that found at day 20 after surgery. Similarly, an upregulation of BDNF mRNA levels was observed in ipsilateral trigeminal ganglion only one day after CCI-ION (Figure 6B). However, no significant change in BDNF mRNA levels was noted in ipsilateral Sp5C in CCI-ION- compared to sham-rats one day after surgery, like that already noted at day 20 post-CCI (Figure 6B).

These data suggest that BDNF expression was differentially induced in central tissues at cephalic versus extra-cephalic levels after peripheral nerve ligation. Accordingly, it could be hypothesized that BDNF overexpression contributed to pain signalling sensitization at the spinal level (in agreement with Merighi et al., 2008; Wang et al., 2009) but not in Sp5c.

### Comparative Example 6

### Effects of acute treatment with tapentadol on mechanical allodynia induced by intrathecal administration of BDNF in healthy rats

In a first part of this study, BDNF (0.3 ng in 25 µl of saline per rat) or saline (25 µl) was injected intrathecally in adult male rats slightly anesthetized with isoflurane (according to Mestre et al. (1994), J. Pharmacol. Toxicol. Meth., 32, 197-200), and animals were subjected to von Frey filaments test applied to hindpaws at various times thereafter.

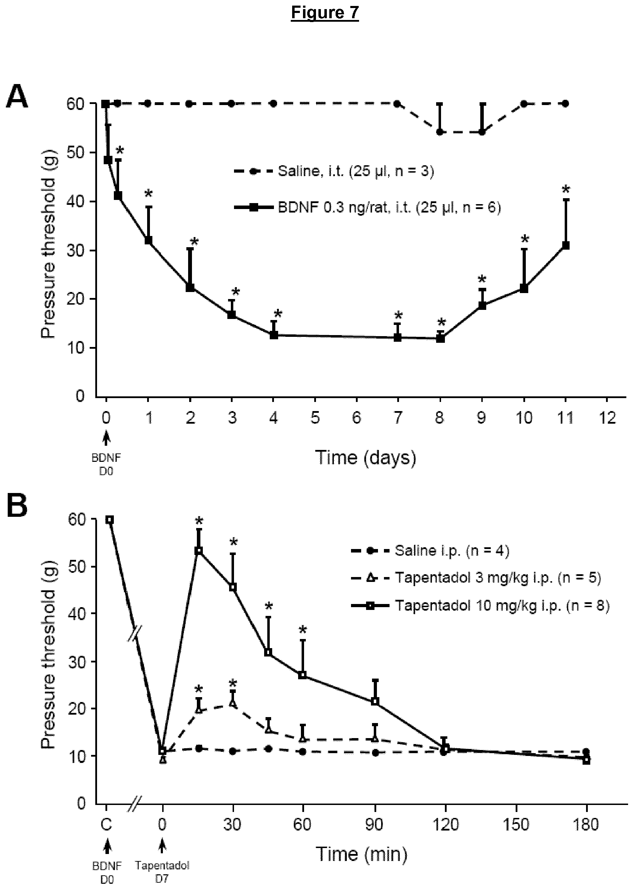

The results are summarized and depicted in Figure 7A. Pressure threshold values are the means ± S.E.M. of the number of independent determinations indicated in parentheses.

* P < 0.05 compared to pressure threshold values determined in the same rats before anesthesia for intrathecal injection (0 on abscissa) ; Dunnett's test.

In becomes evident from Figure 7A that a unique intrathecal injection of BDNF led to a progressive and long lasting decrease in pressure threshold value to trigger hindpaw withdrawal in the von Frey filament test. Thus, from day 4 to day 8 after this treatment, pressure threshold values were as low as those previously determined two weeks after surgery in CCI-SN rats (see Figure. 2). Thereafter, pressure threshold values progressively increased, but they were still only half of those found in untreated healthy rats on the 11th day after the acute intrathecal injection of BDNF (Figure 7A). In contrast, pressure threshold values to trigger nocifensive responses to von Frey filaments applied within the vibrissal pad were not significantly modified at any time up to 11 days after intrathecal administration of BDNF (threshold values remained stable, close to 12 g, like that found in intact healthy rats; data not shown). Accordingly, it can be assumed that i.t. injected BDNF did not diffuse at supraspinal sites, or that this neurotrophic factor does not cause allodynia at cephalic level.

In a second part of this study, tapentadol (3 or 10 mg/kg i.p.) or saline was aministered to rats on the 7th day (Time = 0 on abscissa) after intrathecal injection of BDNF (Time = C) as described above. Pressure threshold values were determined using von Frey filaments test applied to hindpaws.

The results are summarized and depicted in Figure 7B. Each point is the mean ± S.E.M. of the number of independent determinations indicated in parentheses.

* P < 0.05 compared to pressure threshold values determined just prior to Tapentadol or saline injection (0 on abscissa) ; Dunnett's test.

As shown in Figure 7B, tapentadol reversed BDNF-induced allodynia in a dose-dependent manner, with the 10 mg/kg i.p. dose allowing return of pressure threshold values up to levels determined in untreated healthy rats (C on abscissa) at 15-30 min after injection. Thereafter, the anti-allodynic effect of Tapentadol progressively vanished following a time course resembling that previously observed in CCI-SN rats (see Figure 2).

### Comparative Example 7

### Effects of reboxetine compared to tapentadol on mechanical allodynia induced by intrathecal administration of BDNF (A) or chronic constriction injury to the sciatic nerve (B)

In a first part of this study (A), reboxetine (10 mg/kg i.p.; mesylate, Ascent Scientific, Bristol, UK), tapentadol (10 mg/kg i.p.) or saline (0.5 ml i.p./rat) was injected on the 7th day after intrathecal administration of BDNF (0.3 ng in 25 µl of saline per rat ). Pressure threshold values were determined at various times thereafter using von Frey filaments test applied to hindpaws.

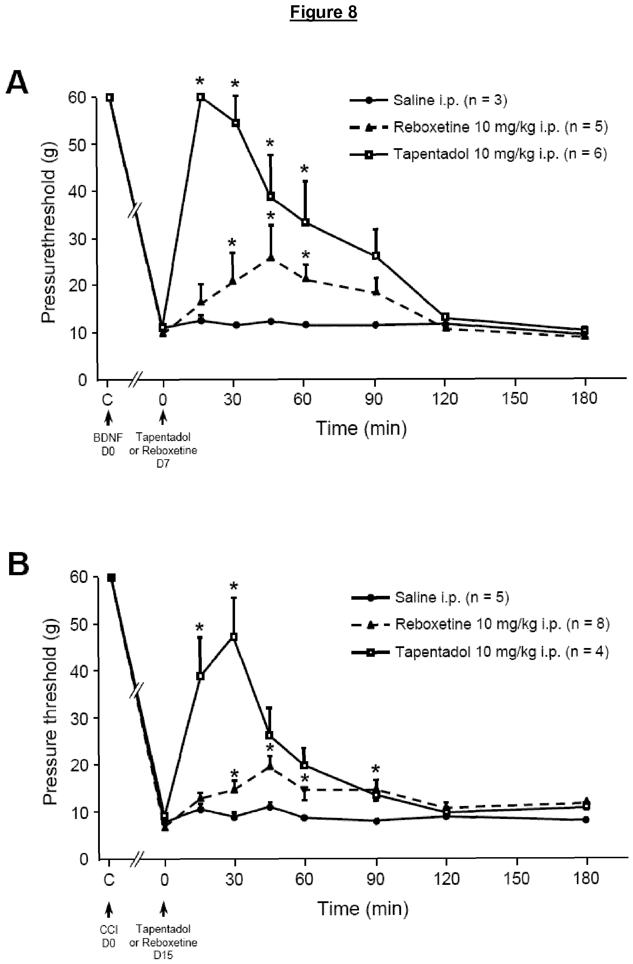

The results are summarized and depicted in Figure 8A. Each point is the mean ± S.E.M. of the number of independent determinations indicated in parentheses.

* P < 0.05 compared to pressure threshold values determined just prior to reboxetine, tapentadol or saline injection (0 on abscissa) ; Dunnett's test.

As shown in Figure 8A, acute administration of reboxetine produced a significant but partial reversal of mechanical allodynia caused by a unique intrathecal injection of BDNF (0.3 ng in 25 µl of saline) 7 days before. At its maximum, reboxetine-induced increase in pressure threshold values in the von Frey filament test was only one third of that caused by 10 mg/kg i.p. of Tapentadol (Figure 8A). Furthermore, the effect of reboxetine developed relatively slowly compared to that of tapentadol because the maximal increase caused by these drugs was reached at 45 min and 15 min, respectively (Figure 8A).

In a second part of this study (B), unilateral chronic constriction injury to the sciatic nerve was performed two weeks before acute treatment with reboxetine (10 mg/kg i.p.), tapentadol (10 mg/kg i.p.) or saline (0.5 ml i.p./rat). Pressure threshold values were then determined at various times using von Frey filaments test applied to ipsilateral hindpaw.

The results are summarized and depicted in Figure 8B. Each point is the mean ± S.E.M. of the number of independent determinations indicated in parentheses.

* P < 0.05 compared to pressure threshold values determined just prior to reboxetine, tapentadol or saline injection (0 on abscissa) ; Dunnett's test.

As shown in Figure 8B, a significant but only partial reversal of CCI-SN-induced decrease in pressure threshold values was noted after acute systemic administration of reboxetine (10 mg/kg i.p.) two weeks after surgery. Interestingly, like that found in unoperated rats which received an intrathecal injection of BDNF (Figure 8A), the anti-allodynic effect of reboxetine developed more slowly and was much less pronounced than that evoked by tapentadol (10 mg/kg i.p.) in CCI-SN rats (Figure 8B).

### Comparative Example 8

### Effects of combined acute treatment with reboxetine and morphine on mechanical allodynia in CCI-SN rats

Before studying the effects of combined acute treatment with reboxetine and morphine on mechanical allodynia in CCI-SN rats, the dose-dependent effect of acute treatment with morphine on mechanical allodynia in CCI-SN rats was determined first.

### Treatment with morphine

Morphine (1, 3 and 10 mg/kg s.c.) or its vehicle (0.9% NaCl) was injected acutely 14 days after CCI-SN surgery. Sham-operated rats were treated in parallel. Pressure threshold values to trigger nocifensive responses to von Frey filaments application onto the plantar surface of ipsilateral hindpaw were determined at various times after acute injection of morphine or saline.

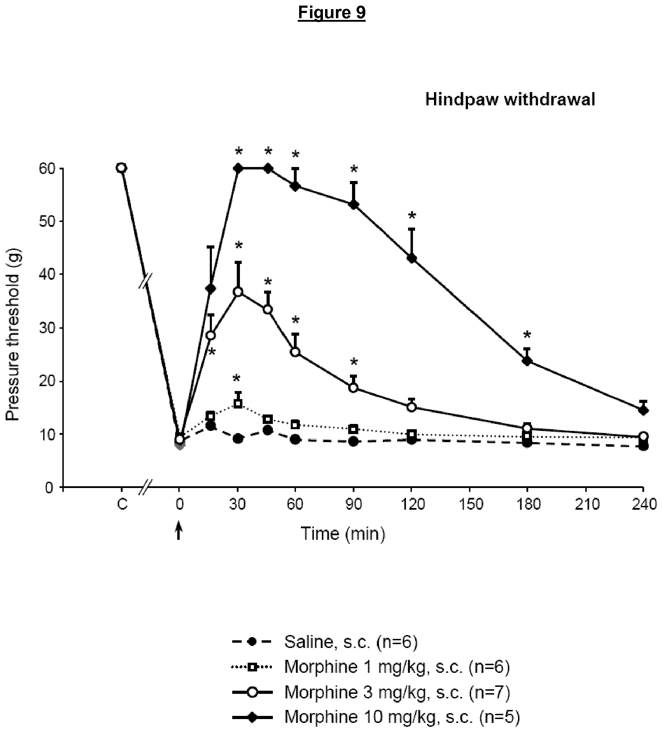

The results are summarized and depicted in Figure 9. Each point is the mean ± S.E.M. of 5-7 independent determinations (as indicated in parentheses). * P < 0.05 compared to pressure threshold values determined in CCI-SN rats just prior to morphine or saline injection (arrow, 0 on abscissa); Dunnett's test.

As shown in Figure 9, morphine at the dose of 10 mg/kg s.c. fully reversed CCI-SN-induced mechanical allodynia as soon as 30 min after the drug injection, and this effect persisted for at least one hour. At the other two doses tested, 1 and 3 mg/kg s.c., allodynia was not completely reversed by morphine and the drug effect was of shorter duration. Thus, these latter two doses were selected for investigating whether or not reboxetine could promote the effect of morphine.

### Treatment with reboxetine and morphine at low dose

14 days after CCI-SN surgery, rats were injected with reboxetine (10 mg/kg i.p.) or its vehicle (0.9% NaCl) followed, 15 min later, by morphine (1 mg/kg s.c.) or its vehicle (0.9% NaCl) and subjected to von Frey filament tests for the following 4 hours. Sham-operated rats were treated in parallel.

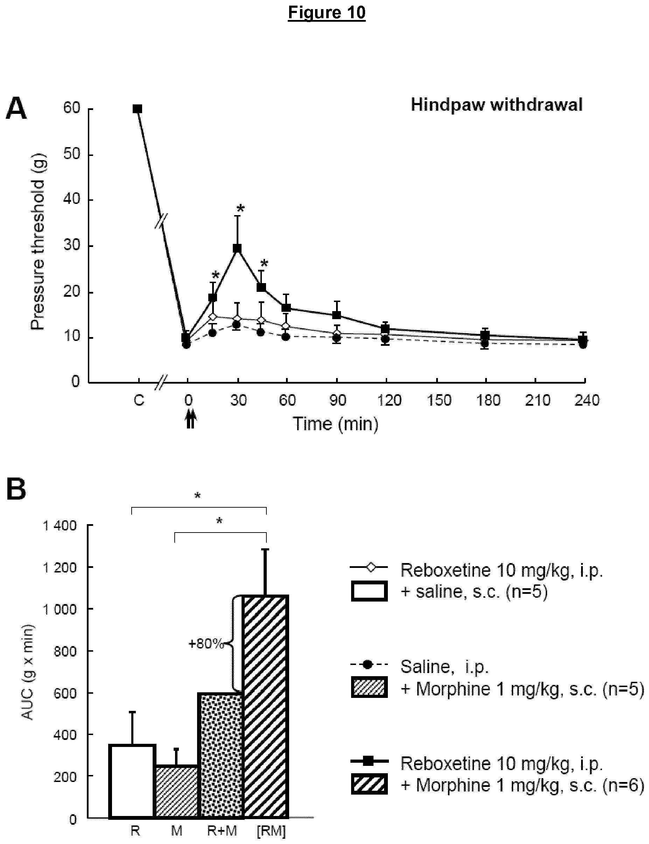

The resulting time-course curves of the threshold values are depicted in Figure 10A. Each point is the mean ± S.E.M. of 5-6 independent determinations (as indicated in parentheses). * P < 0.05 compared to pressure threshold values determined in CCI-SN rats just prior to the second injection (arrow, 0 on abscissa); Dunnett's test.

As shown in Figure 10A, only discrete increases in pressure threshold values were noted after the administration of either drug alone. In contrast, a marked increase was noted after the combined treatment indicated a clear-cut antiallodynic effect of reboxetine + morphine in CCI-SN rats.

Calculation (according to the trapezoidal rule) of respective areas under the time-course curves (AUC values) yielded for the reboxetine+morphine combination [RM] a value 80% higher than the sum [R+M] of the respective values for reboxetine or morphine administered alone, indicating that the resulting antiallodynic effect of the drug combination largely exceeded that expected from simple addition of the effects of each drug considered separately (cf. Figure 10B).

### Treatment with reboxetine and morphine at intermediate dose

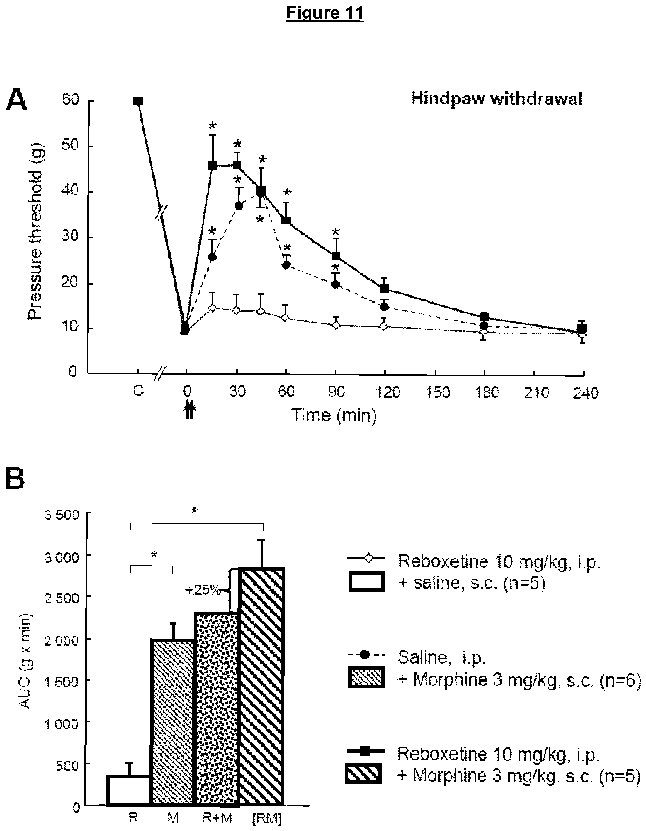

Whether such an apparent synergy between reboxetine and morphine may also occur at the intermediate dose of morphine, 3.0 mg/kg s.c., was tested under the same time conditions as before but in other groups of CCI-SN rats. The results are depicted in Figure 11.

It becomes evident from Figure 11A that the combined treatment with reboxetine + morphine was more effective than either drug alone in increasing pressure the threshold value to trigger hindpaw withdrawal in the von Frey filaments test. However, calculation of the corresponding AUC values indicated that the overall anti-allodynic effect of the combination of reboxetine + morphine [RM] was only 25% higher (P > 0.05) than the sum of those induced by each drug administered separately [R+M], (cf. Figure 11B). Accordingly, with 3.0 mg/kg s.c. of morphine, no real synergy between the anti-allodynic effect of this opiate agonist and that of reboxetine (10 mg/kg i.p.) could be evidenced.

### Example 9

### Effects of combined acute treatment with reboxetine and morphine on mechanical allodynia in CCI-ION rats

For this last series of experiments aimed at investigating whether a synergy between the anti-allodynic effects of reboxetine and morphine might also occur at the cephalic level, in CCI-ION rats, morphine was used at the dose of 3 mg/kg s.c. (intermediate dose of Example 8).

14 days after CCI-ION surgery, rats were injected with reboxetine (10 mg/kg i.p.) or its vehicle (0.9% NaCl) followed, 15 min later, by morphine (3 mg/kg s.c.) or its vehicle (0.9% NaCl) and subjected to von Frey filament tests for the following 4 hours. Sham-operated rats were treated in parallel.

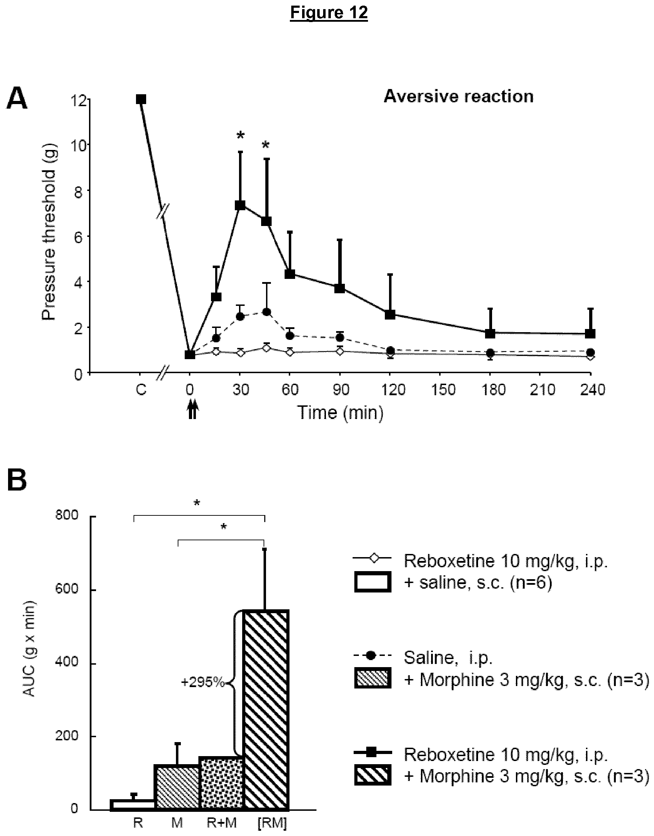

The resulting time-course curves of the threshold values are depicted in Figure 12A. Each point is the mean ± S.E.M. of 3-6 independent determinations (as indicated in parentheses). * P < 0.05 compared to pressure threshold values determined in CCI-SN rats just prior to the second injection (arrow, 0 on abscissa); Dunnett's test.

Upon comparison of Figures 11 and 12, it becomes evident that although this dose was strongly anti-allodynic in CCI-SN rats (see Example 8 and Figure 11A), morphine produced only a discrete effect in CCI-ION rats. At its maximum, the resulting increase in pressure threshold value only reached 20% of that determined in healthy intact rats (Figure 12A). On the other hand, reboxetine (10 mg/kg i.p.) was completely inactive (Figure 12A).

In contrast, the combination of reboxetine and morphine exerted a clear-cut anti-allodynic effect, which was considerably higher than that evoked by either drug alone. Indeed, AUC values corresponding to individual time-course curves indicated that the overal effect of the combination of reboxetine + morphine [RM] was 295% higher than the sum of the effects of each drug administered separately [R+M] (Figure 12B).

The latter data strongly suggest that the synergy between the µ-opiod receptor agonist morphine and the NA reuptake inhibitor reboxetine was not only present but was even more pronounced in CCI-ION rats compared to CCI-SN rats.

This synergy observed between reboxetine and morphine suggests that the mixed inhibition of noradrenaline reuptake and activation of µ-opioid receptors achieved with the single molecule of tapentadol contribute to the remarkable anti-neuropathic pain efficacy of this drug, in particular with regard to the treatment of central neuropathic pain.

## Claims

1. Tapentadol for use in the treatment of pain associated with a disorder of the trigeminal nerve, which is formulated for oral administration.

2. Tapentadol for use according to claim 1, which is solid.

3. Tapentadol for use according to any one of the preceding claims, wherein the pain is moderate to severe.

4. Tapentadol for use according to any one of the preceding claims, which is provided in form of a pharmaceutical dosage form.

5. Tapentadol for use according to claim 4, wherein the pharmaceutical dosage form is a tablet.

6. Tapentadol for use according to claim 4 or 5, wherein the pharmaceutical dosage form is adapted for administration twice daily (*bid*).

7. Tapentadol for use according to any of claims 4 to 6, wherein the pharmaceutical dosage form contains tapentadol in an amount of 10 to 300 mg.

## Patentansprüche

1. Tapentadol zur Verwendung bei der Behandlung von Schmerzen, die einer Erkrankung des Trigeminusnervs zugeordnet sind, das zur oralen Verabreichung formuliert ist.

2. Tapentadol zur Verwendung nach Anspruch 1, welches fest ist.

3. Tapentadol zur Verwendung nach einem der vorhergehenden Ansprüche, wobei der Schmerz mäßig bis stark ist.

4. Tapentadol zur Verwendung nach einem der vorhergehenden Ansprüche, welches in Form einer pharmazeutischen Darreichungsform bereitgestellt wird.

5. Tapentadol zur Verwendung nach Anspruch 4, wobei die pharmazeutische Darreichungsform eine Tablette ist.

6. Tapentadol zur Verwendung nach Anspruch 4 oder 5, wobei die pharmazeutische Darreichungsform zur Verabreichung zweimal täglich angepasst ist *(bid).*

7. Tapentadol zur Verwendung nach einem der Ansprüche 4 bis 6, wobei die pharmazeutische Darreichungsform Tapentadol in einer Menge von 10 bis 300 mg enthält.

## Revendications

1. Tapentadol destiné à être utilisé dans le traitement des douleurs associées à un trouble du nerf trijumeau, qui est formulé pour une administration orale.

2. Tapentadol destiné à être utilisé selon la revendication 1, qui est solide.

3. Tapentadol destiné à être utilisé selon l'une quelconque des revendications précédentes, dans lequel les douleurs vont de modérées à sévères.

4. Tapentadol destiné à être utilisé selon l'une quelconque des revendications précédentes, qui est fourni sous une forme galénique pharmaceutique.

5. Tapentadol destiné à être utilisé selon la revendication 4, dans lequel la forme galénique pharmaceutique est un comprimé.

6. Tapentadol destiné à être utilisé selon la revendication 4 ou 5, dans lequel la forme galénique pharmaceutique est adaptée pour être administrée deux fois par jour *(bid).*

7. Tapentadol destiné à être utilisé selon l'une quelconque des revendications 4 à 6, dans lequel la forme galénique pharmaceutique contient du tapentadol en une quantité de 10 à 300 mg.
